# EUROPEAN PATENT SPECIFICATION

(11) **EP 3 525 708 B1**
(45) Date of publication and mention of the grant of the patent: **01.01.2020**
(21) Application number: 17783864.6
(22) Date of filing: 13.10.2017
(51) Int. Cl.: A61B 18/20, A61B 18/14, A61B 18/00

(54) **A HAIR CUTTING DEVICE AND A METHOD OF OPERATING A HAIR CUTTING DEVICE**
HAARSCHNEIDEVORRICHTUNG UND VERFAHREN ZUM BETRIEB EINER HAARSCHNEIDEVORRICHTUNG
DISPOSITIF DE COUPE DE CHEVEUX ET PROCÉDÉ DE FONCTIONNEMENT D'UN DISPOSITIF DE COUPE DE CHEVEUX

(30) Priority: 14.10.2016 EP 16194005
(43) Date of publication of application: 21.08.2019
(73) Proprietor: Koninklijke Philips N.V., 5656 AE Eindhoven (NL)
(72) Inventor: MOESKOPS, Bastiaan, Wilhelmus, Maria, 5656 AE Eindhoven (NL); VERHAGEN, Rieko, 5656 AE Eindhoven (NL); BOAMFA, Marius, Iosif, 5656 AE Eindhoven (NL); THUMMA, Kiran, Kumar, 5656 AE Eindhoven (NL); JOHNSON, Mark, Thomas, 5656 AE Eindhoven (NL)
(74) Representative: Uittenbroek, Arie Leendert
(86) International application number: PCT/EP2017/076231
(87) International publication number: WO 2018/069519

(56) References cited:
- EP-A1- 0 906 814
- WO-A1-2014/143670
- US-A1- 2008 244 912
- US-A1- 2009 000 123
- US-A1- 2012 123 444

## Description

### FIELD OF THE INVENTION

The invention relates to a hair cutting device for cutting (e.g. shaving) hair on a body of a subject, and in particular relates to a hair cutting device that uses heat to cut or shave hair and a method of operating a hair cutting device.

### BACKGROUND OF THE INVENTION

Shaving devices for cutting or shaving hair on a body of a subject typically make use of one or more blades that cut hairs as the blade is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular a laser light source is provided that is configured to generate laser light having a wavelength selected to target a predetermined chromophore to effectively cut a hair shaft. A fiber optic is located on a shaving portion of the device that is positioned to receive the laser light from the laser light source at a proximal end, conduct the laser light from the proximal end toward a distal end, and emit the light out of a cutting region of the fiber optic and toward hair when the cutting region is brought in contact with the hair.

To achieve good shaving effectiveness, the cutting element of the shaving device (i.e. the fiber optic in the case of the device in WO 2014/143670) needs to be brought very close to the skin and the laser light needs to have sufficient power to cut the hair through melting. The laser light needs to be able to cut the hair completely to avoid the shaving device pulling the hairs from the follicles, which can cause pain or discomfort (similar to when shaving dry hairs using a conventional bladed shaving device that has a blunt blade).

Another consideration is that when hairs are heated, a smell is produced. This smell can be unpleasant, and the amount of odour is highest when the hair is fully evaporated.

These considerations also apply to shaving devices that use a wire or other type of element that is heated in response to an applied electric current and that heats the hair to effect a cutting action.

Therefore there is a need for an improved hair cutting device that provides good hair cutting effectiveness and that minimises the amount of odour generated during cutting.

### SUMMARY OF THE INVENTION

According to a first aspect, there is provided a hair cutting device for cutting hair on a body of a subject, the hair cutting device comprising a light source for generating light at one or more specific wavelengths corresponding to wavelengths absorbed by one or more chromophores in or on hair; a cutting element that comprises an optical waveguide that is coupled at a first end to the light source to receive light, wherein a portion of a sidewall of the optical waveguide forms a cutting face for contacting hair; a sensor for measuring a force acting on the optical waveguide; and a control unit that is coupled to the light source and the sensor, wherein the control unit is configured to control the power of the light generated by the light source based on the measured force, and wherein the power of the light is controlled to a first non-zero power level if the measured force is below a first threshold, and controlled to a second non-zero power level if the measured force is above a second threshold, wherein the first non-zero power level is less than the second non-zero power level and the first threshold is equal to or less than the second threshold.

In some embodiments, the first threshold and the second threshold have the same value. In other embodiments, the first threshold and the second threshold have different values. In the latter embodiments, the control unit can be further configured to control the power of the light to an intermediate non-zero power level if the measured force is more than the first threshold and less than the second threshold, wherein the intermediate non-zero power level is more than the first power level and less than the second power level.

In some embodiments, the control unit is configured to stop the light source generating light or control the light source to generate light at a power level below the first power level if the measured force is less than a third threshold, wherein the third threshold is less than the first threshold.

In some embodiments, the sensor is for measuring the force acting on the optical waveguide in a horizontal direction that is parallel to the direction of movement of the hair cutting device when the hair cutting device is used to cut hair, and wherein the control unit is configured to control the power of the light generated by the light source to the first non-zero power level if the measured force in the horizontal direction is above the third threshold.

In some embodiments, the sensor is for measuring the force acting on the optical waveguide in a vertical direction that is perpendicular to the direction of movement of the hair cutting device when the hair cutting device is used to cut hair and perpendicular to an optical axis of the optical waveguide, and wherein the control unit is configured to control the power of the light generated by the light source to the second non-zero power level if the measured force in the vertical direction is above the second threshold.

In some embodiments, the first power level is a power level that induces the melting of hair when the light couples from the optical waveguide into the hair.

According to a second aspect, there is provided a method of operating a hair cutting device to cut hair on a body of a subject, the hair cutting device comprising a cutting element that comprises an optical waveguide, wherein a portion of a sidewall of the optical waveguide forms a cutting face for contacting hair, the method comprising measuring a force acting on the optical waveguide; and controlling the power of light generated by a light source that is coupled to the optical waveguide based on the measured force, wherein the power of the light is controlled to a first non-zero power level if the measured force is below a first threshold, and controlled to a second non-zero power level if the measured force is above a second threshold, wherein the first non-zero power level is less than the second non-zero power level and the first threshold is equal to or less than the second threshold.

In some embodiments, the first threshold and the second threshold have the same value. In other embodiments, the first threshold and the second threshold have different values. In the latter embodiments, the step of controlling the power of light generated by the light source further comprises controlling the power of the light to an intermediate non-zero power level if the measured force is more than the first threshold and less than the second threshold, wherein the intermediate non-zero power level is more than the first power level and less than the second power level.

In some embodiments, the step of controlling the power of light generated by the light source further comprises stopping the light source generating light or controlling the light source to generate light at a power level below the first power level if the measured force is less than a third threshold, wherein the third threshold is less than the first threshold.

In some embodiments, the step of measuring the force comprises measuring the force acting on the optical waveguide in a horizontal direction that is parallel to the direction of movement of the hair cutting device when the hair cutting device is used to cut hair, and wherein the step of controlling the power of light generated by the light source comprises controlling the power of the light generated by the light source to the first non-zero power level if the measured force in the horizontal direction is above the third threshold.

In some embodiments, the step of measuring the force comprises measuring the force acting on the optical waveguide in a vertical direction that is perpendicular to the direction of movement of the hair cutting device when the hair cutting device is used to cut hair and perpendicular to an optical axis of the optical waveguide, and wherein the step of controlling the power of light generated by the light source comprises controlling the power of the light generated by the light source to the second non-zero power level if the measured force in the vertical direction is above the second threshold.

In some embodiments, the first power level is a power level that induces the melting of hair when the light couples from the optical waveguide into the hair.

According to a third aspect, there is provided a computer program product comprising a computer readable medium having computer readable code embodied therein, the computer readable code being configured such that, on execution by a suitable computer, processor or control unit, the computer, processor or control unit is caused to perform any of the methods described above.

According to a fourth aspect, there is provided a hair cutting device for cutting hair on a body of a subject, the hair cutting device comprising a current source for generating an electric current; a heating element that is coupled to the current source to receive the electric current and that is configured to be heated in response to receiving the electric current; a sensor for measuring a force acting on the heating element; and a control unit that is coupled to the current source and the sensor, wherein the control unit is configured to control the level of the current generated by the current source based on the measured force, and wherein the level of the current generated by the current source is controlled to a first non-zero current level if the measured force is below a first threshold, and controlled to a second non-zero current level if the measured force is above a second threshold, wherein the first non-zero current level is less than the second non-zero current level and the first threshold is equal to or less than the second threshold.

In some embodiments, the first threshold and the second threshold have the same value. In other embodiments, the first threshold and the second threshold have different values. In the latter embodiments, the control unit can be further configured to control the level of the current to an intermediate non-zero current level if the measured force is more than the first threshold and less than the second threshold, wherein the intermediate non-zero current level is more than the first current level and less than the second current level.

In some embodiments, the control unit is configured to stop the current source generating current or control the current source to generate current at a current level below the first current level if the measured force is less than a third threshold, wherein the third threshold is less than the first threshold.

In some embodiments, the sensor is for measuring the force acting on the heating element in a horizontal direction that is parallel to the direction of movement of the hair cutting device when the hair cutting device is used to cut hair, and wherein the control unit is configured to control the level of the current generated by the current source to the first non-zero current level if the measured force in the horizontal direction is above the third threshold.

In some embodiments, the sensor is for measuring the force acting on the heating element in a vertical direction that is perpendicular to the direction of movement of the hair cutting device when the hair cutting device is used to cut hair and perpendicular to an axis of the heating element, and wherein the control unit is configured to control the level of the current generated by the current source to the second non-zero current level if the measured force in the vertical direction is above the second threshold.

In some embodiments, the first current level is a current level that heats the heating element to a temperature that induces the melting of hair when the heating element is in contact with hair.

According to a fifth aspect, there is provided a method of operating a hair cutting device to cut hair on a body of a subject, the hair cutting device comprising a heating element that is configured to be heated in response to receiving an electric current, the method comprising measuring a force acting on the heating element; and controlling the level of the current generated by a current source that is coupled to the heating element based on the measured force, wherein the level of the current is controlled to a first non-zero current level if the measured force is below a first threshold, and controlled to a second non-zero current level if the measured force is above a second threshold, wherein the first non-zero current level is less than the second non-zero current level and the first threshold is equal to or less than the second threshold.

In some embodiments, the first threshold and the second threshold have the same value. In other embodiments, the first threshold and the second threshold have different values. In the latter embodiments, the step of controlling the level of current generated by the current source further comprises controlling the level of the current to an intermediate non-zero current level if the measured force is more than the first threshold and less than the second threshold, wherein the intermediate non-zero current level is more than the first current level and less than the second current level.

In some embodiments, the step of controlling the level of current generated by the current source further comprises stopping the current source generating current or controlling the current source to generate current at a level below the first current level if the measured force is less than a third threshold, wherein the third threshold is less than the first threshold.

In some embodiments, the step of measuring the force comprises measuring the force acting on the heating element in a horizontal direction that is parallel to the direction of movement of the hair cutting device when the hair cutting device is used to cut hair, and wherein the step of controlling the level of current generated by the current source comprises controlling the level of the current generated by the current source to the first non-zero current level if the measured force in the horizontal direction is above the third threshold.

In some embodiments, the step of measuring the force comprises measuring the force acting on the heating element in a vertical direction that is perpendicular to the direction of movement of the hair cutting device when the hair cutting device is used to cut hair and perpendicular to an axis of the heating element, and wherein the step of controlling the level of current generated by the current source comprises controlling the level of the current generated by the current source to the second non-zero current level if the measured force in the vertical direction is above the second threshold.

In some embodiments, the first current level is a current level that heats the heating element to a temperature that induces the melting of hair when the heating element is in contact with hair.

According to a sixth aspect, there is provided a computer program product comprising a computer readable medium having computer readable code embodied therein, the computer readable code being configured such that, on execution by a suitable computer, processor or control unit, the computer, processor or control unit is caused to perform any of the methods described above.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to a first embodiment of the invention;
Fig. 2 is a pair of schematic drawings showing different views of an exemplary hair cutting device according to an embodiment of the invention;
Fig. 3 is a graph illustrating the refractive index of hair;
Fig. 4 is an illustration of an optical fibre cutting element;
Fig. 5 is a flow chart illustrating a method of operating a hair cutting device according to the first embodiment;
Fig. 6 is a set of graphs illustrating the control of the power of generated light based on the measured force according to various embodiments;
Fig. 7 is a block diagram of a hair cutting device according to a second embodiment of the invention; and
Fig. 8 is a flow chart illustrating a method of operating a hair cutting device according to the second embodiment.

### DETAILED DESCRIPTION OF THE EMBODIMENTS

The present invention provides an improvement in the cutting effectiveness and minimises the amount of odour generated during cutting by a laser light-based shaving device, for example as described in WO 2014/143670 and a shaving device that uses a heated wire or other heated cutting element to cut hairs. In particular, the invention provides that the power of the laser light (or electric current used to heat the cutting element) is controlled based on the force acting on the cutting element.

The force acting on the cutting element can provide an indication of whether hairs are being cut or pulled by the cutting element, and the power of the laser light or level of the electric current can be controlled to achieve the required cutting effectiveness while minimising the amount of odour. For example, if the measured force indicates that hair is in contact with the cutting element but not being pulled, the power of the laser light or level of the electric current can be controlled to a relatively low level in order to induce melting of the hair. By contrast, if the measured force indicates that hair is being pulled by the cutting element (which will be a much higher force than if hair is being effectively cut while in contact with the cutting element), the power of the laser light or level of the electric current can be controlled to a relatively high level in order to heat the hair more quickly and thus improve the comfort of the hair cutting for the subject, although this quicker cutting is potentially at the expense of increasing the odour produced by the cutting action as the hair will be heated to a higher temperature. In some embodiments, if the measured force indicates that the cutting element is not in contact with hair, the laser light or electric current can be switched off or controlled to a very low level in order to conserve power (which is particularly useful if the hair cutting device is battery-powered). Without the control according to the invention, it would be necessary to set the power to a high level in order to avoid hair pulling and maintain cutting effectiveness, but this power level would produce more odour than if the power level were controlled to the most appropriate level.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

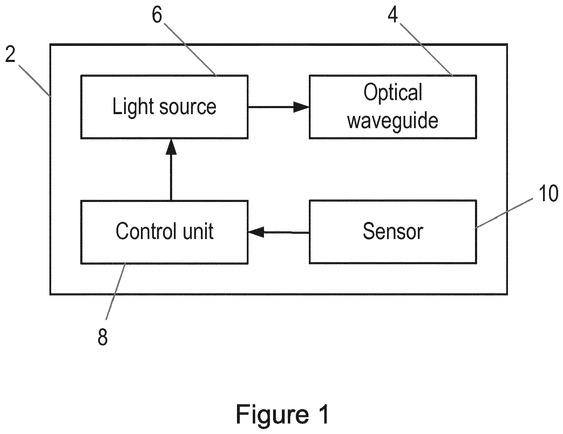

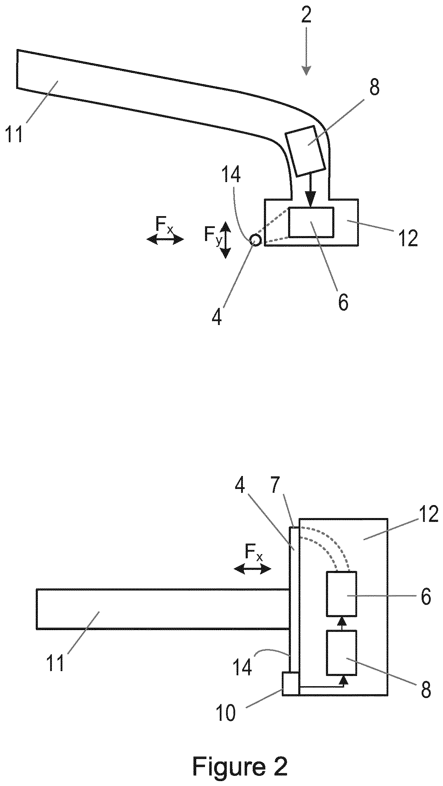

Fig. 1 is a block diagram of a hair cutting device 2 according to a first embodiment of the invention. In this first embodiment, laser light is used to cut hairs. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. In these embodiments, the cutting element 4 is or comprises an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the side wall of the optical waveguide 4 (the side wall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

A light source 6 is provided in the hair cutting device 2 that generates laser light at one or more specific wavelengths. The light source 6 is optically coupled to a first end 7 of the optical waveguide 4 so that the laser light generated by the light source 6 is coupled into the optical waveguide 4 (and specifically coupled into an end of the optical waveguide 4 so that the laser light propagates through the optical waveguide 4). The power (optical power) of the light generated by the light source 6 can be controlled in accordance with embodiments of the invention. In particular the power of the light can be controlled at least between a first power level and a second (higher) power level (with both power levels being non-zero). The change in the power level can be effected by a change in the absolute power level, or, where the light source 6 operates in a pulsed mode of operation and generates pulses of light, the change in power level can be effected by a change in the peak power level and/or by a change in the duty cycle of the light pulse (e.g. by increasing or decreasing the fraction of time that the light is on). As described in more detail below, in some embodiments, the power of the light can be controlled continuously or semi-continuously (e.g. in a step-wise manner) from the first power level to the second power level based on a measured force.

The light source 6 is configured to generate laser light at one or more specific wavelengths that can be used to cut or burn through hair. In particular, each wavelength corresponds to the wavelength of light absorbed by a chromophore that is found in or on hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the light source 6 include, but are not limited to, melanin, keratin and water. Suitable wavelengths of laser light that can be used include, but are not limited to, wavelengths selected from the range 380 nm (nanometers) to 500 nm and 2500 nm to 3500 nm. Those skilled in the art will be aware of the wavelengths of light that are absorbed by these chromophores, and thus also the specific wavelengths of light that the light source 6 should generate for this purpose, and further details are not provided herein.

In some embodiments the light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources 6 can be provided that each generate laser light at a respective wavelength, and each light source 6 can be coupled to a respective optical waveguide 4 to provide multiple cutting elements in the device 2.

The hair cutting device 2 also comprises a control unit 8 that controls the operation of the hair cutting device 2, and in particular is connected to the light source 6 to control the power of the light generated by the light source 6 (for example by outputting a suitable control signal to the light source 6). In some embodiments, the control unit 8 can also control the activation and deactivation of the light source 6, for example in response to an input from a user of the hair cutting device 2 and/or in response to a measured force. The control unit 8 can comprise one or more processors, processing units, multi-core processors or modules that are configured or programmed to control the hair cutting device 2. The control unit 8 can also comprise or be associated with a memory or memory module that stores data and computer readable code that is configured to be executed by the control unit 8 to cause the control unit 8 and hair cutting device 2 to operate according to the embodiments described herein.

In accordance with embodiments of the invention, the hair cutting device 2 also comprises a sensor 10 that measures the force acting on the cutting element (optical waveguide) 4 as the hair cutting device 2 is moved over the skin. A measured force can be due to contact between the optical waveguide 4 and hair, or any other object, such as skin, that the optical waveguide 4 may be moved into contact with. The force represents the force exerted on the optical waveguide 4 by the hair or other object. The sensor 10 provides an output signal representing the measured force to the control unit 8. The control unit 8 can analyse the measured force to determine whether the optical waveguide 4 is effectively cutting hair or whether the optical waveguide 4 is pulling hair.

The sensor 10 can be arranged in the hair cutting device 2 in a number of different ways. In some implementations, the sensor 10 is arranged such that it measures the tension or change in tension in the optical waveguide 4 (e.g. the force along the optical axis of the optical waveguide 4). As hair contacts the optical waveguide 4, the hair will exert a force on the optical waveguide 4 and this will result in an increase in the tension in the optical waveguide 4.

In alternative implementations, the sensor 10 can be arranged in the hair cutting device 2 so that it measures the force acting on the optical waveguide 4 and/or measures a change in force acting on the optical waveguide 4 due to the contact with hair or another object in a direction that is perpendicular to the axis of the optical waveguide 4 and parallel to the direction of movement of the hair cutting device 2 across the skin.

In alternative implementations, the sensor 10 can be arranged in the hair cutting device 2 so that it measures the force or a change in the force acting on the optical waveguide 4 at an angle that is not perpendicular to the axis of the optical waveguide 4 or parallel to the axis of the optical waveguide 4 (i.e. the force is measured at some intermediate angle).

The sensor 10 can be any type of sensor that can be used to measure the force acting on or in the cutting element 4. For example, the sensor 10 can be a strain gauge, a piezoelectric sensor, a sensor based on fibre Bragg gratings.

The sensor 10 can be arranged in the hair cutting device 2 such that it directly measures the force acting on the optical waveguide 4 (e.g. the sensor 10 is in contact with the optical waveguide 4, for example located at an end of the optical waveguide 4 in the case of a sensor 10 that measures the tension or strain in the optical waveguide 4, or located between the optical waveguide 4 and a part of the housing of the hair cutting device 2). Alternatively the sensor 10 can be arranged in the hair cutting device 2 such that it indirectly measures the force acting on the optical waveguide 4, i.e. it measures the force via another part of the housing or mechanism of the hair cutting device 2 that is connected to the optical waveguide 4. In the case of a hair cutting device 2 that has a mechanism for enabling the optical waveguide 4 to move relative to a handle of the hair cutting device 2 and thus follow the contours of the skin, the sensor 10 could be arranged in or near a hinge or other flexible part of the mechanism.

In some embodiments, it may be useful to measure the force exerted by the hair on the optical waveguide 4 in the horizontal direction (referred to as force component Fₓ) and the force exerted by the hair on the optical waveguide 4 in the vertical direction (referred to as force component F_{y}). The horizontal direction is the direction that is parallel to the direction of movement of the hair cutting device 2 when the hair cutting device 2 is used to cut hair. The vertical direction is perpendicular to the direction of movement of the hair cutting device 2 while the hair cutting device 2 is used to cut hair and perpendicular to an axis of the cutting element (e.g. perpendicular to the optical axis of the optical waveguide 4. The force Fₓ is typically associated with contact-forces (i.e. the force of contact between the hair and optical waveguide 4) and cutting forces experienced during efficient hair cutting, and the force F_{y} is typically associated with forces due to pulling of hair (which should ideally be avoided). In some embodiments, a single sensor 10 can be provided to measure both the horizontal and vertical forces, but in other embodiments, multiple sensors 10 can be provided that measure the horizontal and vertical components respectively. The horizontal and vertical directions (associated with the horizontal force component Fₓ and vertical force component F_{y} respectively) are illustrated in Fig. 2 (which is described in more detail below).

It will be appreciated that Fig. 1 only shows the components of the hair cutting device 2 that are required to illustrate and describe the invention, and in a practical implementation the hair cutting device 2 will comprise other components to those shown. For example, the hair cutting device 2 will comprise a power source, such as one or more batteries, or a connector for interfacing with a mains power supply.

As noted above, Fig. 2 shows a hair cutting device 2 that is in the form of a handheld wet razor. Fig. 2 shows a side view and a bottom view of the razor 2. The razor 2 comprises a handle 11 for the subject (or other user of the device 2) to hold, and a head portion 12 that includes the cutting element 4 (optical waveguide/fibre). The sensor 10 is not shown in Fig. 2. As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. A light source 6 and control unit 8 are shown as being incorporated into the head portion 12 and handle 11 respectively, but it will be appreciated that the positions of these components in the hair cutting device 2 as shown in Fig. 2 is not limiting. Likewise it will be appreciated that the embodiment shown in Fig. 2 is merely an example, and the optical waveguide 4, light source 6 and control unit 8 can be incorporated or used in place of a conventional blade in any type of hair cutting device 2 that conventionally comprises a blade for physically cutting or slicing hair (whether the blade is static or actuated in order to achieve a cutting action).

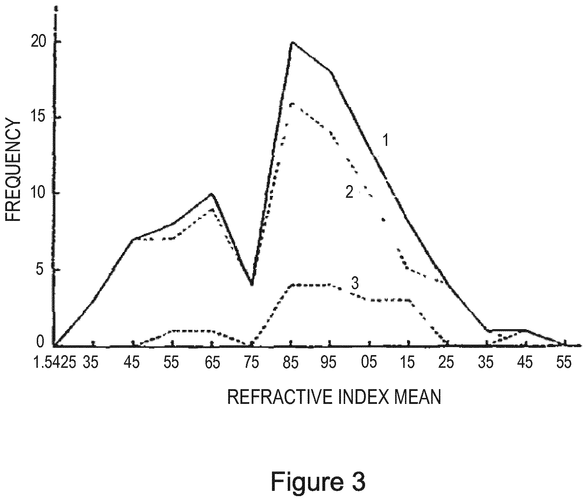

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light source 6 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 must have the same or a lower refractive index than hair at the point at which the hair contacts the optical waveguide 4. Thus, the optical waveguide 4 must have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair. Light may still be able to couple from the optical waveguide 4 into an object (e.g. a hair) brought into contact with the cutting face 14 of the optical waveguide 4 even if the refractive index of the optical waveguide 4 is higher than that of the object due to a high numerical aperture in the cutting face.

Thus, in some embodiments, the refractive index of the optical waveguide 4 at least at the cutting face 14 is equal to or lower than 1.56. More preferably the refractive index of the optical waveguide 4 at least at the cutting face 14 is equal to or lower than 1.55. Even more preferably, the refractive index of the optical waveguide 14 at least at the cutting face 14 is equal to or lower than 1.54, since this refractive index is below the refractive indices identified in Fig. 3.

In some embodiments, a lower bound for the refractive index of the optical waveguide 4 at the cutting face 14 can be 1.48, 1.51, 1.53 or 1.54.

A range of values from which the refractive index of the optical waveguide 4 is selected can be formed from any combination of the upper and lower refractive index bounds set out in the preceding paragraphs.

The optical waveguide/fibre 4 can be made from any suitable material or combination of materials. For example optical waveguides/fibres can be composed of or comprise silica, fluoride glass, phosphate glass, chalcogenide glass, and/or crown glass (such as BK7), and/or crystals such as sapphire, yttrium aluminium garnet (YAG), etc.

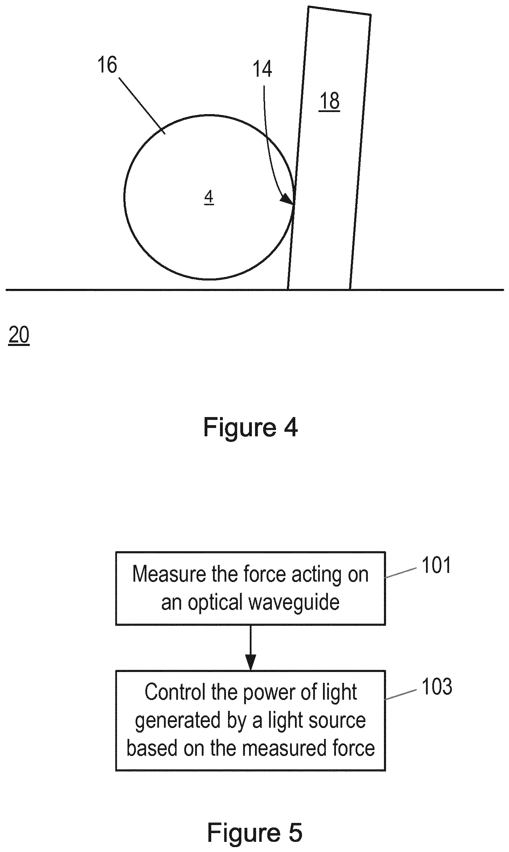

Fig. 4 illustrates an exemplary embodiment of the optical waveguide 4. The optical waveguide 4 is for use in or with a hair cutting device 2, for example as shown in Figs. 1 and 2. In Fig. 4 the optical waveguide 4 is shown side on (i.e. looking down the optical axis of the optical waveguide 4), and no other support element for the optical waveguide 4 is shown. It will be appreciated that Fig. 4 is not necessarily drawn to scale.

In Fig. 4, the optical waveguide 4 has a core 16. In this illustrated embodiment, the optical waveguide 4 does not include any cladding around the core 16. However it will be appreciated that in some embodiments the optical waveguide 4 can comprise cladding around the core 16, although preferably no cladding is present along the cutting face 14 (and indeed, in some embodiments the cutting face 14 can correspond to those parts of the optical waveguide 4 where there is no cladding).

The optical waveguide 4 is shown in contact with a hair 18 and close to, but not in contact with, the skin 20. The portion of the side wall of the core 16/optical waveguide 4 that is intended to contact hairs during use forms the cutting face 14. As described above, the refractive index of the core 16 is the same or lower than the refractive index of hair.

The core 16 may have a uniform refractive index (i.e. the same refractive index throughout the core 16), or it may be a graded index fibre, which means that the refractive index decreases with increasing distance from the optical axis.

The flow chart in Fig. 5 illustrates a method of operating a hair cutting device 2 to cut hair on a body of a subject (e.g. hair on the head, face, neck, torso, arms or legs) according to the first embodiment. In a first step, step 101, the force acting on the optical waveguide 4 is measured. The force can be measured by sensor 10. As noted above, when the optical waveguide 4 is in contact with hair, the measured force is indicative of whether the optical waveguide 4 is effectively cutting the hair or whether the optical waveguide 4 is pulling the hair.

Next, in step 103, the power of the light generated by the light source 6 is controlled based on the measured force. Step 103 can be performed by the control unit 8 controlling the light source 6

Step 103 can comprise controlling the power of the light to a first power level that induces melting of hair when that light couples out of the optical waveguide 4 and into hair if the measured force indicates that hair is in contact with the optical waveguide 4, and controlling the power of the light to a second (higher) power level if the measured force indicates that hair is being pulled, or even epilated (i.e. pulled out) by the optical waveguide 4. Thus, if the measured force indicates that hair is being pulled by the optical waveguide 4, the power level of the generated light is increased in order to heat the hair more quickly (and thus cut the hair more quickly). Of course, it will be appreciated that this higher power level may result in the hair being evaporated, and thus generating more odour, but this is preferable to the subject experiencing the pulling of hairs by the optical waveguide 4.

In practice, to maintain cutting effectiveness while minimising the odour generated by the heating of hairs, step 103 can comprise the control unit 8 controlling the power of the generated light to a first power level if the measured force is below a first threshold, and controlling the power of the generated light to a second power level if the measured force is above a second threshold. Both the first power level and the second power level are non-zero (i.e. neither power level corresponds to the light source 6 being switched off or deactivated), the second power level is higher than the first power level and the value of the first threshold is equal to or less than the value of the second threshold.

The values of the first threshold and the second threshold are set to distinguish between forces consistent with hair being in contact with the optical waveguide 4 (but being cut effectively), and forces consistent with hair being pulled by the optical waveguide 4. The force on an optical waveguide 4 when hair is being pulled is higher than the force on an optical waveguide 4 when hair is being cut effectively. In some cases, a force around 0.1 Newtons (N) can be indicative of hair being in contact with the optical waveguide 4 but being cut effectively, and a force above 0.5 N can be indicative of hair being pulled by the optical waveguide 4.

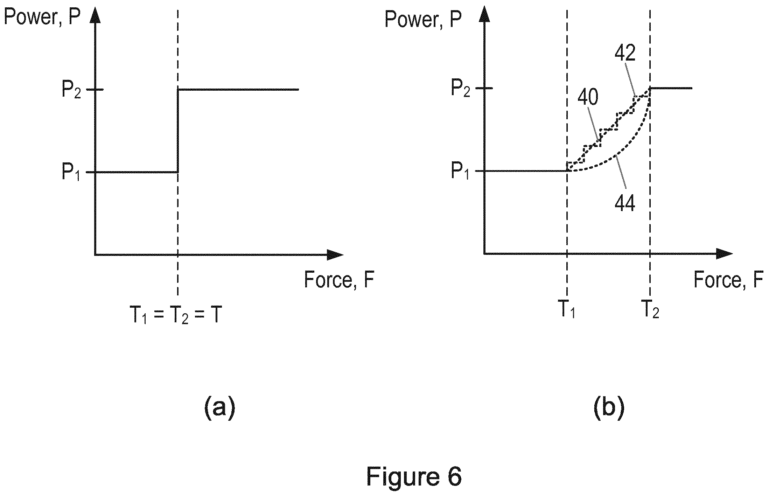

The graph in Fig. 6(a) illustrates a first implementation of step 103. Fig. 6(a) shows the power of the generated light, P, for a given measured force, F, on the optical waveguide 4. In this implementation, the first threshold (indicated by T₁) and the second threshold (indicated by T₂) have the same value (i.e. T₁ = T₂ = T), and thus when the measured force is less than the threshold T (i.e. F < T₁ = T), the power P is set to a first power level P₁. In some implementations, the value of T can be between 0.1 N and 0.5 N (i.e. 0.1 N ≤ T ≤ 0.5 N). The first power level P₁ is sufficient to heat hair to the point of melting as the optical waveguide 4 is drawn across the skin and into contact with hairs, but is not too high that it heats the hair to the point of evaporation (thereby minimising the odour generated). In some embodiments, the first power level is the minimum power level (or at least the minimum power level) required to heat hair to the point of melting. The minimum power level required to heat hair to the point of melting depends on the diameter of the optical waveguide 4 and/or the material the optical waveguide 4 is formed from or formed of. An exemplary value for the first power level (for an optical waveguide 4 with a specific diameter and material) is 150 milliWatts (mW).

However, if the measured force is above the threshold T (i.e. F > T₁ = T), indicative of the hair being pulled by the optical waveguide 4, the power P is set to a second power level P₂ (that is greater than the first power level P₁). The second power level P₂ is a power level that will heat the hair more quickly, and thus cut the hair more quickly, thereby reducing the pulling of the hair by the optical waveguide 4. In some embodiments, the second power level P₂ corresponds to a maximum possible power level or maximum permitted power level for the generated light, although power levels below these maxima can be used if required. An exemplary value for the second power level is 500 mW (for an optical waveguide 4 with a specific diameter and material).

Fig. 6(b) shows an alternative implementation of step 103 in which the first threshold and the second threshold have different values. As in Fig. 6(a), the graph in Fig. 6(b) shows the power of the generated light, P, for a given measured force, F, on the optical waveguide 4. In this implementation, the first threshold (indicated by T₁) and the second threshold (indicated by T₂) have different values (i.e. T₁ ≠ T₂), and the first threshold is less than the second threshold (T₁ < T₂). In some examples, the first threshold can have a value at or around 0.1 N, and the second threshold can have a value at or around 0.5 N (although it will be appreciated that other values can be used). When the measured force is less than the first threshold T₁ (i.e. F < T₁), the power P is set to the first power level P₁ (similar to the implementation shown in Fig. 6(a)). Thus the first power level P₁ is sufficient to heat hair to the point of melting as the optical waveguide 4 is drawn across the skin and into contact with hairs, but is not too high that it heats the hair to the point of evaporation (thereby minimising the odour generated). An exemplary value for the first power level is 150 mW (although as noted above this value is dependent on the characteristics of the optical waveguide 4). In some embodiments, the first power level is the minimum power level (or at least the minimum power level) required to heat hair to the point of melting.

If the measured force is above the second threshold T₂ (i.e. F > T₂) the power P is set to the second power level P₂. As in Fig. 6(a), the second power level P₂ is a power level that will heat the hair more quickly, and thus cut the hair more quickly, thereby reducing the pulling of the hair by the optical waveguide 4. An exemplary value for the second power level is 500 mW (although as noted above this value is dependent on the characteristics of the optical waveguide 4). In some embodiments, the second power level P₂ corresponds to a maximum possible power level or maximum permitted power level for the generated light (or some power level below either of these maxima).

As the first threshold and the second threshold are different values, in this implementation there is an intermediate region where the force on the optical waveguide 4 is between the first threshold and the second threshold (i.e. T₁ < F < T₂). The force on the optical waveguide 4 can depend on the number of hairs contacting the optical waveguide 4 at any given time and/or the rate at which the optical waveguide 4 is cutting through the hairs, and thus it is possible for the force on the optical waveguide 4 to increase beyond the first threshold in the case of a denser region of hair (i.e. more hairs per unit area of the skin), without the hairs being pulled (or at least without the subject noticing any significant pulling of hairs). However, in this region the cutting effectiveness may be slightly or significantly decreased (e.g. depending on how much denser the hair is), so in this implementation the power of the generated light is set higher than the first power level in the intermediate region (but less than the second power level), with the specific power level being based on the measured force. Thus, the power of the light generated by the light source 6 can be set to one or more intermediate (non-zero) power levels based on the measured force. As shown in Fig. 6(b), the power level can be increased in a linear manner (as shown by straight line 40), in a stepped manner (as shown by stepped line 42) or in a non-linear manner (as shown by curved line 44). In some embodiments, when the measured force is in the intermediate region, the control unit 8 can input the measured force into a mathematical function that determines a power level to use. Alternatively, the control unit 8 can maintain or store a look-up table that maps measured force to power level. It will be appreciated that the stepped manner 42 can be implemented by comparing the measured force to a number of additional thresholds having values between the first threshold and the second threshold.

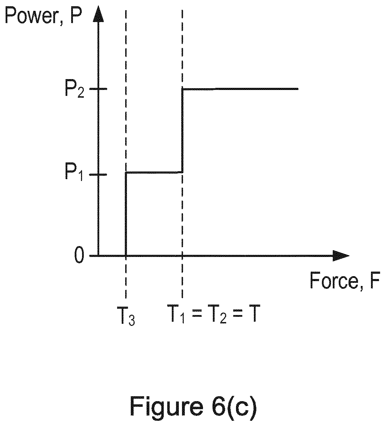

In some embodiments, the light source 6 can be controlled to stop generating light if the measured force indicates that the optical waveguide 4 is not in contact with hair. This can help to conserve power, and is particularly useful if the hair cutting device 2 is battery-powered. This is shown in Fig. 6(c), which corresponds to the embodiment shown in Fig. 6(a), with the application of an additional threshold, T₃. Thus, if the measured force is below the third threshold, T₃ (which is lower than the first threshold T₁), then the power of the generated light can be set to zero (i.e. the light is switched off), or, alternatively, the light source 6 can be put into a low power or power saving mode where the power of the generated light is not sufficient to melt hairs. Where the first threshold is at or around 0.1 N, an exemplary value for the third threshold T₃ can be 0.05 N. It will be appreciated that the third threshold T₃ could also be applied to the embodiment shown in Fig. 6(b).

As noted above, where the light source 6 generates continuous light, the control unit 8 can change the power level by changing the power (e.g. increasing or decreasing the power). Where the light source 6 operates in a pulsed mode of operation and generates pulses of light, the control unit 8 can control the light source 6 to change the power level by changing the peak power level (e.g. by increasing or decreasing the peak power) and/or by changing the duty cycle of the light pulse (e.g. by increasing or decreasing the fraction of time that the light is on).

Preferably steps 101 and 103 are repeated at a relatively high frequency (e.g. 10 kHz) in order to make sure that the power of the generated light is adapted quickly to any changes in the measured force. It will be appreciated that the frequency with which step 103 is performed will be less than the sampling frequency of the sensor 10 (where the sampling frequency is the frequency with which the sensor 10 measures the force).

As noted above, in some embodiments, the sensor 10 may be able to separately measure the horizontal and vertical components of the force acting on the optical waveguide 4. The horizontal component is indicative of the contact force with hair and the cutting forces experienced during efficient hair cutting (this component is indicated by the arrow labelled Fₓ in Fig. 2). The vertical component is typically associated with the pulling of hair by the optical waveguide 4 and should be avoided (this component is indicated by the arrow labelled F_{y} in Fig. 2). In this embodiment, the horizontal component can be compared to a threshold (e.g. the third threshold described above) to determine whether to operate with a power level that induces melting of hair (e.g. the first power level described above) or to stop generating light (or to generate light at a power below that required to melt hair. The vertical component can be compared to a threshold (e.g. the first threshold or second threshold described above) to determine whether the optical waveguide 4 is pulling hairs rather than cutting, and if the vertical component is above the threshold, the power of the generated light can be set at the second power level in order to cut the hairs more quickly. Thus, as an example, before the horizontal force reaches the horizontal component threshold the light power can be set to an energy-saving level (e.g. zero), and after the horizontal force reaches the horizontal component threshold the light power can be set to the first power level in order to cut hair with a minimum of evaporation and odour, and after the vertical force component exceeds the vertical component threshold the light power can be set to the second power level in order to cut hair more quickly and minimise the pulling forces.

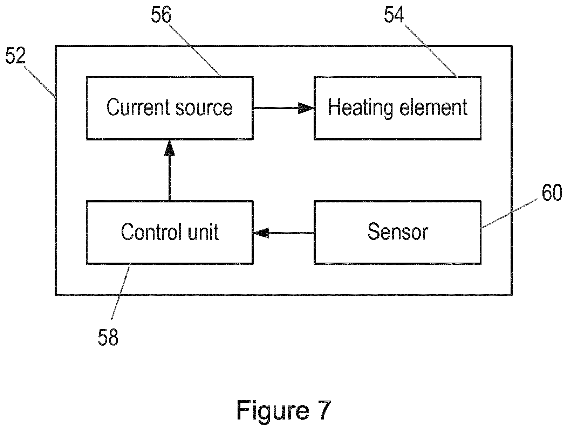

As noted above, the present invention can also be applied to hair cutting devices that use a heated wire or other heated cutting element to cut hairs. Fig. 7 is a block diagram of a hair cutting device 52 according to a second embodiment of the invention. As with the first embodiment, the hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 52 comprises a cutting element 54 that enables hair to be cut as the hair cutting device 52 is moved over the skin of a subject. In these embodiments, the cutting element 54 is or comprises an elongate heating element (such as a wire or filament) that is arranged on the hair cutting device 52 so that the axis of the heating element 54 is generally perpendicular to the direction in which the hair cutting device 52 is moved so that hairs contact the side wall of the heating element 54 as the hair cutting device 52 is moved across the skin of the subject.

A current source 56 is provided in the hair cutting device 52 that generates electrical current. The current source 56 is coupled to the heating element 54 so that the current generated by the current source 56 is coupled into the heating element 54. Current flowing through the heating element 54 causes the heating element 54 to heat up, and this heated is transferred to any hair in contact with the heating element 54, causing the hair to melt (if sufficient heat is transferred). The current generated by the current source 56 can be controlled in accordance with embodiments of the invention. In particular the current can be controlled at least between a first current level and a second (higher) current level (with both current levels being non-zero). In some embodiments, the current can be controlled continuously or semi-continuously (e.g. in a step-wise manner) from the first current level to the second current level based on a measured force.

The hair cutting device 52 also comprises a control unit 58 that controls the operation of the hair cutting device 52, and in particular is connected to the current source 56 to control the current generated by the current source 56. In some embodiments, the control unit 58 can also control the activation and deactivation of the current source 56, for example in response to an input from a user of the hair cutting device 52 and/or in response to a measured force. The control unit 58 can be implemented and used to control the current output by the current source 56 in response to a measured force in a similar way to the control unit 8 in the embodiment of Fig. 1 is used to control the power of the light generated by the light source 6 in response to the measured force, and thus the details of the control unit 8 and its operation described above apply also to the control unit 58 of Fig. 7.

The hair cutting device 52 also comprises a sensor 60 that measures the force acting on the cutting element (heating element) 54 as the hair cutting device 52 is moved over the skin. The sensor 60 can be implemented and used in a similar way to the sensor 10 in the embodiment of Fig. 1, and thus the details of the sensor 10 provided above apply also to the sensor 60 of Fig. 7 (including the embodiment where the horizontal and vertical components of the contact force are measured separately).

It will be appreciated that Fig. 7 only shows the components of the hair cutting device 52 that are required to illustrate and describe the invention, and in a practical implementation the hair cutting device 52 will comprise other components to those shown. For example, the hair cutting device 52 will comprise a power source, such as one or more batteries, or a connector for interfacing with a mains power supply.

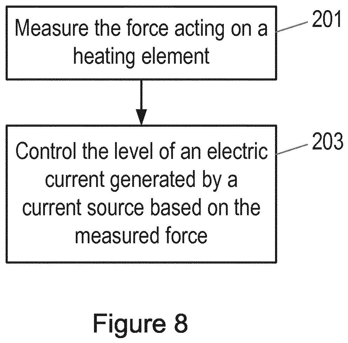

Fig. 8 is a flow chart that illustrates a method of operating the hair cutting device 52 according to the second embodiment to cut hair on a body of a subject (e.g. hair on the head, face, neck, torso, arms or legs). In a first step, step 201, the force acting on the heating element 54 is measured. The force can be measured by sensor 60. When the heating element 54 is in contact with hair, the measured force is indicative of whether the heating element 54 is effectively cutting the hair or whether the heating element 54 is pulling the hair.

Next, in step 203, the current generated by the current source 56 is controlled based on the measured force. Step 203 can be performed by the control unit 58 controlling the current source 56.

Step 203 can comprise controlling the current to a first current level that causes the heating element 54 to heat to a temperature that induces melting of hair when hair is in contact with the heating element 54 if the measured force indicates that hair is in contact with the heating element 54, and controlling the current to a second (higher) current level if the measured force indicates that hair is being pulled, or even epilated (i.e. pulled out) by the heating element 54. Thus, if the measured force indicates that hair is being pulled by the heating element 54, the current level is increased in order to increase the temperature of the heating element 54 and thus heat the hair more quickly (and therefore cut the hair more quickly). Of course, it will be appreciated that this higher current level may result in the hair being evaporated, and thus generating more odour, but this is preferable to the subject experiencing the pulling of hairs by the heating element 54.

As in the first embodiment described above, in practice, to maintain cutting effectiveness while minimising the odour generated by the heating of hairs, step 203 can comprise the control unit 58 controlling the current to a first current level if the measured force is below a first threshold, and controlling the current to a second current level if the measured force is above a second threshold. Both the first current level and the second current level are non-zero (i.e. neither current level corresponds to the current source 56 being switched off or deactivated), the second current level is higher than the first current level and the value of the first threshold is equal to or less than the value of the second threshold.

The values of the first threshold and the second threshold are set to distinguish between forces consistent with hair being in contact with the heating element 54 (but being cut effectively), and forces consistent with hair being pulled by the heating element 54. The force on a heating element 54 when hair is being pulled is higher than the force on a heating element 54 when hair is being cut effectively. In some cases, a force around 0.1 N can be indicative of hair being in contact with the heating element 54 and being cut effectively, and a force above 0.5 N can be indicative of hair being pulled by the heating element 54.

It will be appreciated that the implementations of step 103 shown in the graphs of Fig. 6 for the control of the light level based on the measured force can be applied to implementations of step 203 for the control of the current level based on the measured force.

There is therefore provided an improved hair cutting device that maintains hair cutting effectiveness while avoiding the evaporation of hair.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A hair cutting device for cutting hair on a body of a subject, the hair cutting device comprising:
a source for generating light at one or more specific wavelengths corresponding to wavelengths absorbed by one or more chromophores in or on hair;
a cutting element that comprises an optical waveguide that is coupled at a first end to the source to receive light, wherein a portion of a sidewall of the optical waveguide forms a cutting face for contacting hair;
a sensor for measuring a force acting on the cutting element; and
a control unit that is coupled to the source and the sensor, wherein the control unit is configured to control the power of the light generated by the source based on the measured force, and wherein the power of the light is controlled to a first non-zero level if the measured force is below a first threshold, and controlled to a second non-zero level if the measured force is above a second threshold, wherein the first non-zero level is less than the second non-zero level and the first threshold is equal to or less than the second threshold.

2. A hair cutting device for cutting hair on a body of a subject, the hair cutting device comprising:
a source for generating an electric current;
a cutting element that comprises a heating element that is coupled to the source to receive the electric current and that is configured to be heated in response to receiving the electric current;
a sensor for measuring a force acting on the cutting element; and
a control unit that is coupled to the source and the sensor, wherein the control unit is configured to control the level of the current generated by the source based on the measured force, and wherein the level of the current generated by the source is controlled to a first non-zero level if the measured force is below a first threshold, and controlled to a second non-zero level if the measured force is above a second threshold, wherein the first non-zero level is less than the second non-zero level and the first threshold is equal to or less than the second threshold.

3. A hair cutting device as claimed in claim 1 or 2, wherein the first threshold and the second threshold have the same value.

4. A hair cutting device as claimed in claim 1 or 2, wherein the first threshold and the second threshold have different values.

5. A hair cutting device as claimed in claim 4, wherein the control unit is further configured to control the level generated by the source to an intermediate non-zero level if the measured force is more than the first threshold and less than the second threshold, wherein the intermediate non-zero level is more than the first level and less than the second level.

6. A hair cutting device as claimed in any of claims 1-5, wherein the control unit is configured to stop the source generating or control the source to generate at a level below the first level if the measured force is less than a third threshold, wherein the third threshold is less than the first threshold.

7. A hair cutting device as claimed in any of claims 1-6, wherein the sensor is for measuring the force acting on the cutting element in a horizontal direction that is parallel to the direction of movement of the hair cutting device when the hair cutting device is used to cut hair, and wherein the control unit is configured to control the level generated by the source to the first non-zero level if the measured force in the horizontal direction is above the third threshold.

8. A hair cutting device as claimed in any of claims 1-7, wherein the sensor is for measuring the force acting on the cutting element in a vertical direction that is perpendicular to the direction of movement of the hair cutting device when the hair cutting device is used to cut hair and perpendicular to an axis of the cutting element, and wherein the control unit is configured to control the level generated by the source to the second non-zero level if the measured force in the vertical direction is above the second threshold.

9. A hair cutting device as claimed in any of claims 1-8, wherein the first level is a level that induces the melting of hair.

10. A method of operating a hair cutting device to cut hair on a body of a subject, the hair cutting device comprising a cutting element that comprises an optical waveguide, wherein a portion of a sidewall of the optical waveguide forms a cutting face for contacting hair, the method comprising:
measuring a force acting on the cutting element; and
controlling the power of light generated by a source that is coupled to the optical waveguide based on the measured force, wherein the power of the light is controlled to a first non-zero level if the measured force is below a first threshold, and controlled to a second non-zero level if the measured force is above a second threshold, wherein the first non-zero level is less than the second non-zero level and the first threshold is equal to or less than the second threshold.

11. A method of operating a hair cutting device to cut hair on a body of a subject, the hair cutting device comprising a cutting element that comprises a heating element that is configured to be heated in response to receiving an electric current, the method comprising:
measuring a force acting on the cutting element; and
controlling the level of the current generated by a source that is coupled to the heating element based on the measured force, wherein the level of the current is controlled to a first non-zero level if the measured force is below a first threshold, and controlled to a second non-zero level if the measured force is above a second threshold, wherein the first non-zero level is less than the second non-zero level and the first threshold is equal to or less than the second threshold.

12. A method as claimed in claim 10 or 11, wherein the first threshold and the second threshold have the same value.

13. A method as claimed in claim 10 or 11, wherein the first threshold and the second threshold have different values.

14. A method as claimed in claim 10, wherein the first power level is a power level that induces the melting of hair when the light couples from the optical waveguide into the hair.

15. A computer program product comprising a computer readable medium having computer readable code embodied therein, the computer readable code being configured such that, on execution by a suitable computer, processor or control unit, the computer, processor or control unit is caused to perform the method of any of claims 10-14.

## Patentansprüche

1. Haarschneidevorrichtung zum Schneiden von Haaren an einem Körper eines Subjekts, wobei die Haarschneidevorrichtung umfasst:
eine Quelle zum Erzeugen von Licht auf einer oder mehreren spezifischen Wellenlängen, die Wellenlängen entsprechen, die von einem oder mehreren Chromophoren in oder auf dem Haar absorbiert werden;
ein Schneidelement, das einen optischen Wellenleiter umfasst, der an einem ersten Ende mit der Quelle gekoppelt ist, um Licht zu empfangen, wobei ein Abschnitt einer Seitenwand des optischen Wellenleiters eine Schneidfläche zum In-Kontakt-Treten mit dem Haar bildet;
einen Sensor zum Messen einer Kraft, die auf das Schneidelement einwirkt; und
eine Regeleinheit, die mit der Quelle und dem Sensor gekoppelt ist, wobei die Regeleinheit konfiguriert ist, um die Leistung des Lichts, das von der Quelle erzeugt wird, basierend auf der gemessenen Kraft zu regeln, und wobei die Leistung des Lichts auf einen ersten Pegel ungleich null geregelt wird, falls die gemessene Kraft unterhalb eines ersten Schwellenwerts liegt, und auf einen zweiten Pegel ungleich null geregelt wird, falls die gemessene Kraft oberhalb eines zweiten Schwellenwerts liegt, wobei der erste Pegel ungleich null kleiner als der zweite Pegel ungleich ist und der erste Schwellenwert gleich oder kleiner als der zweite Schwellenwert ist.

2. Haarschneidevorrichtung zum Schneiden von Haaren an einem Körper eines Subjekts, wobei die Haarschneidevorrichtung umfasst:
eine Quelle zum Erzeugen eines elektrischen Stroms;
ein Schneidelement, das ein Heizelement umfasst, das mit der Quelle gekoppelt ist, um den elektrischen Strom zu empfangen, und das konfiguriert ist, um als Reaktion auf das Empfangen des elektrischen Stroms erwärmt zu werden;
einen Sensor zum Messen einer Kraft, die auf das Schneidelement einwirkt; und
eine Regeleinheit, die mit der Quelle und dem Sensor gekoppelt ist, wobei die Regeleinheit konfiguriert ist, um den Strompegel, der von der Quelle erzeugt wird, basierend auf der gemessenen Kraft zu regeln, und wobei der Strompegel, der von der Quelle erzeugt wird, auf einen ersten Pegel ungleich null geregelt wird, falls die gemessene Kraft unterhalb eines ersten Schwellenwerts liegt, und auf einen zweiten Pegel ungleich null geregelt wird, falls die gemessene Kraft oberhalb eines zweiten Schwellenwerts liegt, wobei der erste Pegel ungleich null kleiner als der zweite Pegel ungleich ist und der erste Schwellenwert gleich oder kleiner als der zweite Schwellenwert ist.

3. Haarschneidevorrichtung nach Anspruch 1 oder 2, wobei der erste Schwellenwert und der zweite Schwellenwert den gleichen Wert aufweisen.

4. Haarschneidevorrichtung nach Anspruch 1 oder 2, wobei der erste Schwellenwert und der zweite Schwellenwert unterschiedliche Werte aufweisen.

5. Haarschneidevorrichtung nach Anspruch 4, wobei die Regeleinheit weiter konfiguriert ist, um den Pegel, der von der Quelle erzeugt wird, auf einen Zwischenpegel ungleich null zu regeln, falls die gemessene Kraft größer als der erste Schwellenwert und kleiner als der zweite Schwellenwert ist, wobei der Zwischenpegel ungleich null größer als der erste Pegel und kleiner als der zweite Pegel ist.

6. Haarschneidevorrichtung nach einem der Ansprüche 1-5, wobei die Regeleinheit konfiguriert ist, um das Quellenerzeugen zu stoppen oder um die Quelle zu regeln, um bei einem Pegel unterhalb des ersten Pegels zu erzeugen, falls die gemessene Kraft kleiner als ein dritter Schwellenwert ist, wobei der dritte Schwellenwert kleiner als der erste Schwellenwert ist.

7. Haarschneidevorrichtung nach einem der Ansprüche 1-6, wobei der Sensor zum Messen der Kraft dient, die auf das Schneidelement in einer horizontalen Richtung einwirkt, die parallel zur Bewegungsrichtung der Haarschneidevorrichtung ist, wenn die Haarschneidevorrichtung verwendet wird, um Haare zu schneiden, und wobei die Regeleinheit konfiguriert ist, um den Pegel zu regeln, der von der Quelle auf den ersten Pegel ungleich null geregelt wird, falls die gemessene Kraft in der horizontalen Richtung oberhalb des dritten Schwellenwertes ist.

8. Haarschneidevorrichtung nach einem der Ansprüche 1-7, wobei der Sensor zum Messen der Kraft dient, die auf das Schneidelement in einer vertikalen Richtung einwirkt, die senkrecht zu der Bewegungsrichtung der Haarschneidevorrichtung ist, wenn die Haarschneidevorrichtung verwendet wird, um Haare zu schneiden, und senkrecht zu einer Achse des Schneidelements, und wobei die Regeleinheit konfiguriert ist, um den Pegel, der von der Quelle erzeugt wird, auf den zweiten Pegel ungleich null zu regeln, falls die gemessene Kraft in der vertikalen Richtung oberhalb des zweiten Schwellenwertes ist.

9. Haarschneidevorrichtung nach einem der Ansprüche 1-8, wobei der erste Pegel ein Pegel ist, der das Schmelzen von Haar einleitet.

10. Verfahren zum Betreiben einer Haarschneidevorrichtung zum Schneiden von Haaren an einem Körper eines Subjekts, wobei die Haarschneidevorrichtung ein Schneidelement umfasst, das einen optischen Wellenleiter umfasst, wobei ein Abschnitt einer Seitenwand des optischen Wellenleiters eine Schneidfläche zum In-Kontakt-Treten mit dem Haar bildet, das Verfahren umfassend:
Messen einer Kraft, die auf das Schneidelement einwirkt; und
Regeln der Leistung von Licht, das durch eine Quelle erzeugt wird, die mit dem optischen Wellenleiter gekoppelt ist, basierend auf der gemessenen Kraft, wobei die Leistung des Lichts auf einen ersten Pegel ungleich null geregelt wird, falls die gemessene Kraft unterhalb eines ersten Schwellenwerts liegt, und auf einen zweiten Pegel ungleich null geregelt wird, falls die gemessene Kraft oberhalb eines zweiten Schwellenwerts liegt, wobei der erste Pegel ungleich null kleiner als der zweite Pegel ungleich null ist und der erste Schwellenwert gleich oder kleiner als der zweite Schwellenwert ist.

11. Verfahren zum Betreiben einer Haarschneidevorrichtung zum Schneiden von Haaren an einem Körper eines Subjekts, wobei die Haarschneidevorrichtung ein Schneidelement umfasst, das eine Heizelement umfasst, das konfiguriert ist, um als Reaktion auf das Empfangen eines elektrischen Stroms erwärmt zu werden, das Verfahren umfassend:
Messen einer Kraft, die auf das Schneidelement einwirkt; und
Regeln des Strompegels, der durch eine Quelle erzeugt wird, die mit dem Heizelement gekoppelt ist, basierend auf der gemessenen Kraft, wobei der Strompegel auf einen ersten Pegel ungleich null geregelt wird, falls die gemessene Kraft unterhalb eines ersten Schwellenwerts liegt, und auf einen zweiten Pegel ungleich null geregelt wird, falls die gemessene Kraft oberhalb eines zweiten Schwellenwerts liegt, wobei der erste Pegel ungleich null kleiner als der zweite Pegel ungleich ist und der erste Schwellenwert gleich oder kleiner als der zweite Schwellenwert ist.

12. Verfahren nach Anspruch 10 oder 11, wobei der erste Schwellenwert und der zweite Schwellenwert den gleichen Wert aufweisen.

13. Verfahren nach Anspruch 10 oder 11, wobei der erste Schwellenwert und der zweite Schwellenwert unterschiedliche Werte aufweisen.

14. Verfahren nach Anspruch 10, wobei der erste Leistungspegel ein Leistungspegel ist, der das Schmelzen von Haaren einleitet, wenn das Licht vom optischen Wellenleiter in das Haar eingekoppelt wird.

15. Computerprogrammprodukt, umfassend ein computerlesbares Medium, das computerlesbaren Code darin ausgeführt aufweist, wobei der computerlesbare Code derart konfiguriert ist, dass bei Ausführung durch einen geeigneten Computer, Prozessor oder Regeleinheit der Computer, Prozessor oder die Regeleinheit veranlasst werden, das Verfahren nach einem der Ansprüche 10-14 durchzuführen.

## Revendications

1. Dispositif de coupe de cheveux pour couper des cheveux sur le corps d'un sujet, le dispositif de coupe de cheveux comprenant :
une source pour générer de la lumière à une ou plusieurs longueurs d'ondes spécifiques correspondant aux longueurs d'ondes absorbées par un ou plusieurs chromophores dans ou sur les cheveux ;
un élément de coupe qui comprend un guide d'onde optique qui est couplé à une première extrémité à la source pour recevoir de la lumière, dans lequel une partie d'une paroi latérale du guide d'onde optique forme une face de coupe pour venir en contact avec les cheveux ;
un capteur pour mesurer une force agissant sur l'élément de coupe ; et
une unité de commande qui est couplée à la source et au capteur, dans lequel l'unité de commande est configurée pour commander la puissance de la lumière générée par la source sur la base de la forme mesurée et dans lequel la puissance de la lumière est commandée à un premier niveau non nul si la force mesurée se situe en dessous d'un premier seuil et commandée à un deuxième niveau non nul si la force mesurée se situe au-dessus d'un deuxième seuil, dans lequel le premier niveau non nul est inférieur au deuxième niveau non nul et le premier seuil est égal ou inférieur au deuxième seuil.

2. Dispositif de coupe de cheveux pour couper les cheveux sur le corps d'un sujet, le dispositif de coupe de cheveux comprenant :
une source pour générer un courant électrique ;
un élément de coupe qui comprend un élément chauffant qui est couplé à la source pour recevoir le courant électrique et qui est configuré pour être chauffé en réponse à la réception du courant électrique ;
un capteur pour mesurer une force agissant sur l'élément de coupe ; et
une unité de commande qui est couplée à la source et au capteur, dans lequel l'unité de commande est configurée pour commander le niveau du courant généré par la source sur la base de la force mesurée et dans lequel le niveau du courant généré par la source est commandé à un premier niveau non nul si la force mesurée se situe en dessous d'un premier seuil et commandé à un deuxième niveau non nul si la force mesurée se situe au-dessus d'un deuxième seuil, dans lequel le premier niveau non nul est inférieur au deuxième niveau non nul et le premier seuil est égal ou inférieur au deuxième seuil.

3. Dispositif de coupe de cheveux selon la revendication 1 ou 2, dans lequel le premier seuil et le deuxième seuil ont la même valeur.

4. Dispositif de coupe de cheveux selon la revendication 1 ou 2, dans lequel le premier seuil et le deuxième seuil ont des valeurs différentes.

5. Dispositif de coupe de cheveux selon la revendication 4, dans lequel l'unité de commande est en outre configurée pour commander le niveau généré par la source à un niveau non nul intermédiaire si la force mesurée est supérieure au premier seuil et inférieure au deuxième seuil, dans lequel le niveau non nul intermédiaire est supérieur au premier niveau et inférieur au deuxième niveau.

6. Dispositif de coupe de cheveux selon l'une quelconque des revendications 1-5, dans lequel l'unité de commande est configurée pour arrêter la source génératrice ou commander la source pour générer à un niveau en dessous du premier niveau si la force mesurée est inférieure à un troisième seuil, dans lequel le troisième seuil est inférieur au premier seuil.

7. Dispositif de coupe de cheveux selon l'une quelconque des revendications 1-6, dans lequel le capteur est destiné à mesurer la force agissant sur l'élément de coupe dans une direction horizontale qui est parallèle à la direction de déplacement du dispositif de coupe de cheveux lorsque le dispositif de coupe de cheveux est utilisé pour couper les cheveux et dans lequel l'unité de commande est configurée pour commander le niveau généré par la source au premier niveau non nul si la force mesurée dans la direction horizontale se situe au-dessus du troisième seuil.

8. Dispositif de coupe de cheveux selon l'une quelconque des revendications 1-7, dans lequel le capteur est destiné à mesurer la force agissant sur l'élément de coupe dans une direction verticale qui est perpendiculaire à la direction de déplacement du dispositif de coupe de cheveux lorsque le dispositif de coupe de cheveux est utilisé pour couper des cheveux et perpendiculaire à l'axe de l'élément de coupe et dans lequel l'unité de commande est configurée pour commander le niveau généré par la source au deuxième niveau non nul si la force mesurée dans la direction verticale se situe au-dessus du deuxième seuil.

9. Dispositif de coupe de cheveux selon l'une quelconque des revendications 1-8, dans lequel le premier niveau est un niveau qui induit la fusion des cheveux.

10. Procédé d'exploitation d'un dispositif de coupe de cheveux pour couper des cheveux sur le corps d'un sujet, le dispositif de coupe de cheveux comprenant un élément de coupe qui comprend un guide d'onde optique, dans lequel une partie d'une paroi latérale du guide d'onde optique forme une face de coupe pour venir en contact avec les cheveux, le procédé comprenant :
la mesure d'une force agissant sur l'élément de coupe ; et
la commande de la puissance de la lumière générée par une source qui est couplée au guide d'onde optique sur la base de la force mesurée, dans lequel la puissance de la lumière est commandée à un premier niveau non nul si la force mesurée se situe en dessous d'un premier seuil et commandée à un deuxième niveau non nul si la force mesurée se situe au-dessus d'un deuxième seuil, dans lequel le premier niveau non nul est inférieur au deuxième niveau non nul et le premier seuil est égal ou inférieur au deuxième seuil.

11. Procédé d'exploitation d'un dispositif de coupe de cheveux pour couper des cheveux sur le corps d'un sujet, le dispositif de coupe de cheveux comprenant un élément de coupe qui comprend un élément chauffant qui est configuré pour être chauffé en réponse à la réception d'un courant électrique, le procédé comprenant :
la mesure d'une force agissant sur l'élément de coupe ; et
la commande du niveau du courant généré par une source qui est couplée à l'élément chauffant sur la base de la force mesurée, dans lequel le niveau du courant est commandé à un premier niveau non nul si la force mesurée se situe en dessous d'un premier seuil et commandé à un deuxième niveau non nul si la force mesurée se situe au-dessus d'un deuxième seuil, dans lequel le premier niveau non nul est inférieur au deuxième niveau non nul et le premier seuil est égal ou inférieur au deuxième seuil.

12. Procédé selon la revendication 10 ou 11, dans lequel le premier seuil et le deuxième seuil ont la même valeur.

13. Procédé selon la revendication 10 ou 11, dans lequel le premier seuil et le deuxième seuil ont différentes valeurs.

14. Procédé selon la revendication 10, dans lequel le premier niveau de puissance est un niveau de puissance qui induit la fusion des cheveux lorsque la lumière se couple du guide d'onde optique dans les cheveux.

15. Produit de programme d'ordinateur comprenant un support lisible par ordinateur présentant un code lisible par ordinateur qui y est mis en œuvre, le code lisible par ordinateur étant configuré de sorte que, lors d'une exécution par un ordinateur, un processeur ou une unité de commande appropriée, l'ordinateur, le processeur ou l'unité de commande soit amené à effectuer le procédé selon l'une quelconque des revendications 10-14.
